(19) Europäisches Patentamt
European Patent Office
Office européen des brevets

(11) Veröffentlichungsnummer: **0 108 981**
**B1**

(12) # EUROPÄISCHE PATENTSCHRIFT

(45) Veröffentlichungstag der Patentschrift: **19.02.86**

(51) Int. Cl.⁴: **C 07 D 239/94,** A 61 K 31/505

(21) Anmeldenummer: **83110812.1**

(22) Anmeldetag: **28.10.83**

(54) **Semicarbazide, ihre Herstellung und Verwendung.**

(30) Priorität: **02.11.82 DE 3240456**

(43) Veröffentlichungstag der Anmeldung: **23.05.84 Patentblatt 84/21**

(45) Bekanntmachung des Hinweises auf die Patenterteilung: **19.02.86 Patentblatt 86/8**

(84) Benannte Vertragsstaaten: **AT BE CH DE FR GB IT LI LU NL SE**

(56) Entgegenhaltungen: **DE - A - 2 261 095**

(73) Patentinhaber: **BOEHRINGER INGELHEIM KG, D-6507 Ingelheim am Rhein (DE)**

(72) Erfinder: **Renth, Ernst-Otto, Dr., Frankenstrasse 11, D-6507 Ingelheim am Rhein (DE)**
Erfinder: **Mentrup, Anton, Dr., Steinernstrasse 25, D-6503 Mainz-Kastel (DE)**
Erfinder: **Schromm, Kurt, Dr., In der Dörrwiese 35, D-6507 Ingelheim am Rhein (DE)**
Erfinder: **Traunecker, Werner, Dr., Birkenweg 1, D-6531 Münster-Sarmsheim (DE)**
Erfinder: **Reichl, Richard, Dr., Im Hippel 55, D-6535 Gau-Algesheim (DE)**

**EP 0 108 981 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Semicarbazide, ihre Herstellung nach an sich bekannten Verfahren und ihre Verwendung als therapeutische Wirkstoffe.

Die neuen Verbindungen entsprechen der Formel I

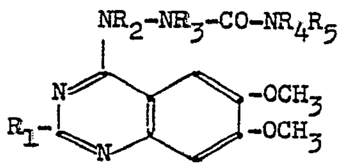

(I)

in der (wie auch in den folgenden Formeln)

$R_1$ Wasserstoff, $C_1$-$C_4$-Alkyl oder Trifluormethyl,

$R_2$, $R_3$ und $R_4$ Wasserstoff, Methyl oder Ethyl,

$R_5$ Wasserstoff, ggf. phenylsubstituiertes $C_1$-$C_8$-Alkyl, $C_3$-$C_7$-Cycloalkyl, $C_1$-$C_4$-Alkoxycarbonylmethyl, Phenyl, Naphthyl, einen durch Methyl, Ethyl, Methoxy, Ethoxy, Trifluormethyl, Fluor, Chlor oder Brom ein- bis dreifach substituierten Phenyl- oder Naphthylrest,

$R_4$ und $R_5$ gemeinsam auch eine ggf. durch O oder $NR_6$ unterbrochene Alkylenkette mit 4 oder 5 Kettengliedern und

$R_6$ ein $C_1$-$C_4$-Alkyl bedeutet.

Sie können als freie Basen oder als Salze mit Säuren vorliegen.

Die Alkylgruppen in den oben definierten Resten können geradkettig oder verzweigt sein. Soweit $R_1$ bis $R_4$ Alkyl bedeutet, handelt es sich in erster Linie um Methyl und Ethyl. Im Falle von $R_5$ enthält der Alkylrest vorzugsweise 3 bis 5 C-Atome, der Cycloalkylrest 5-6 C-Atome, der Alkoxyrest in der Alkoxycarbonylmethylgruppe ist bevorzugt Methoxy oder Ethoxy. Die Substituenten in den aromatischen Resten sind Methyl, Ethyl, Methoxy, Ethoxy, Fluor, Chlor, Brom und $CF_3$. Hervorzuheben sind Methyl, Methoxy und Chlor. Es können bis zu drei gleiche oder verschiedene Substituenten vorhanden sein. Stehen $R_4$ und $R_5$ für eine ggf. durch O oder $NR_6$ unterbrochene Alkylenkette, so stellen sie beispielsweise eine Gruppe wie: $-(CH_2)_4-$

$-(CH_2)_5-$

$-CH_2-CH_2-O-CH_2-CH_2-$ oder

$-CH_2-CH_2-NCH_3-CH_2-CH_2-$ dar.

Der Rest $R_1$ bedeutet vorzugsweise H und $CH_3$. Die Substituenten $R_2$, $R_3$ und $R_4$ stehen vorzugsweise für H.

Die neuen Verbindungen können nach den folgenden Verfahren erhalten werden:

a) Man setzt ein 6,7-Dimethoxychinazolin der Formel II

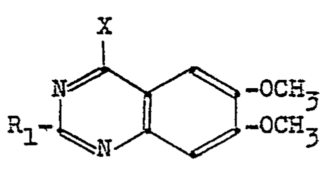

(II)

worin X einen gegen den Stickstoff des Semicarbazids der Formel III

$$HNR_2-NR_3-CO-NR_4R_5 \qquad (III)$$

austauschbaren Rest, vorzugsweise Chlor, bedeutet, mit dem Semicarbazid III in einem unter den Reaktionsbedingungen inerten Lösungsmittel um.

Als Lösungsmittel eignen sich z.B. Dimethylformamid, Acetonitril, Tetrahydrofuran, Alkohole. Die Umsetzung wird je nach der Reaktionsfähigkeit der Komponenten bei Temperaturen zwischen Raumtemperatur und der Siedetemperatur des Reaktionsgemischs durchgeführt, vorzugsweise zwischen 40 und 120° C und in Gegenwart eines basischen Stoffs, z.B. Natriumcarbonat, Kaliumcarbonat, tertiäre Amine.

b) Man setzt ein Chinazolin der Formel IV

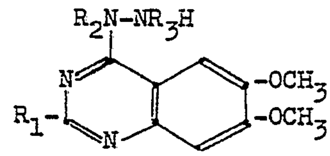

(IV)

mit einem Isocyanat der Formel V

$$OCN-R_5 \qquad (V)$$

in einem unter den Reaktionsbedingungen inerten Lösungsmittel um, wobei Verbindungen der Formel I mit $R_4$ gleich Wasserstoff entstehen.

Die Umsetzung erfolgt in Lösungsmitteln wie Tetrahydrofuran, Dioxan, Ether, Dimethylformamid, Toluol, Aceton, Acetonitril, vorzugsweise bei Raumtemperatur oder unter leichtem Erwärmen. Die Verbindung der Formel IV kann dabei als Suspension vorliegen.

c) Man setzt Verbindungen der Formel IV mit Carbamoylchloriden der Formel VI

$$R''_4R'_5N-COCl \qquad (VI)$$

(worin $R'_4$ und $R'_5$ die gleichen Bedeutungen wie $R_4$ und $R_5$ haben, ausgenommen Wasserstoff) in einem unter den Reaktionsbedingungen inerten Lösungsmittel um. Dabei erhält man Verbindungen der Formel I, in denen $R_4$ und $R_5$ die oben angegebenen Bedeutungen haben, jedoch nicht Wasserstoff sein können.

Für dieses Verfahren eignen sich die bei Verfahren b) erwähnten Lösungsmittel, insbesondere Dimethylformamid. Zweckmässig wird ein säureabfangendes Mittel, etwa Kalium- oder Natriumcarbonat, eine tertiäre organische Base oder Aminüberschuss verwendet.

d) Man setzt eine Verbindung der Formel IV mit einem substituierten Carbaminsäureester der Formel VII

$$R_4R_5-N-COOR \qquad (VII)$$

worin R einen ggf. substituierten gesättigten oder ungesättigten aliphatischen oder araliphatischen Rest bedeutet, bei erhöhter Temperatur in einem unter den Reaktionsbedingungen inerten Lösungsmittel um.

Als Lösungsmittel können beispielsweise Aceton, Dimethylformamid oder Alkohole dienen. Die Reaktionstemperatur liegt im allgemeinen zwischen 40 und 80° C. Bei Verwendung von Lösungsmitteln mit geeignetem Siedepunkt, etwa Methanol, kann bei Rückflusstemperatur gearbeitet werden.

e) Man setzt eine Verbindung der Formel VIII

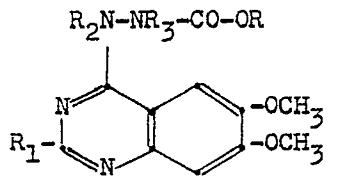

(VIII)

mit einem primären oder sekundären Amin der Formel IX

$$HNR_4R_5 \qquad (IX)$$

bei erhöhter Temperatur in einem unter den Reaktionsbedingungen inerten Lösungsmittel um.

Bei diesem Verfahren können die bei d) genanten Lösungsmittel und Reaktionstemperaturen angewendet werden.

Soweit als Reaktionsprodukte Salze entstehen, können diese gewünschtenfalls in die Basen der Formel I oder in Salze anderer Säuren übergeführt werden. Primär erhaltene Basen können gewünschtenfalls in üblicher Weise in Säureadditionssalze übergeführt werden.

Die Ausgangsstoffe sind bekannt oder können sonst nach üblichen Verfahren erhalten werden.

Die erfindungsgemässem Verbindungen eignen sich als Wirkstoffe für Arzneimittel. Die Verbindungen sind vor allem Phosphordiesterase-Inhibitoren und Herzstimulantien mit langer Wirkungsdauer. Sie erhöhen die Kontraktionskraft des Herzmuskels, ohne die Herzfrequenz wesentlich zu beeinflussen.

Besonders diese Selektivität macht sie für die heilende und prophylaktische Behandlung von pathologischen Herzzuständen, vor allem Herzversagen, brauchbar. Neben der Wirkung auf den Herzmuskel zeigen die erfindungsgemässen Verbindungen noch gefässerweiternde und antihypertensive Eigenschaften, z.T. bewirken sie auch eine Steigerung der Nierendurchblutung und Broncholyse. Die therapeutische und prophylaktische Dosis ist abhängig von der Art und Schwere des Krankheitszustands sowie von der Verabreichungsart.

Bei Verarbreichung an den Menschen liegen die oralen Dosen im Bereich von 20 bis 1000 mg pro Tag, aufgeteilt im allgemeinen auf 2 bis 4 Applikationen. Bei intravenöser Applikation beträgt die Einzeldosis zwischen 1 und 300 mg.

Für die Anwendung eignen sich übliche Zubereitungen, etwa Kapseln, Tabletten, Dragées, Lösungen, Suspensionen für die orale Verabreichung, sterile wässerige isotonische Lösungen für die parenterale Applikation.

Die Arzneimittelzubereitungen enthalten neben den Wirkstoffen gemäss der Erfindung und ggf. weiteren therapeutisch wirksamen Stoffen in der galenischen Pharmazie übliche Hilfs- und/oder Trägerstoffe.

*Beispiele für die Formulierung:*

*Tabletten*

Zusammensetzung:

| | |
|---|---|
| Wirkstoff gemäss der Erfindung | 100 mg |
| Kolloidale Kieselsäure | 10 mg |
| Milchzucker | 118 mg |
| Kartoffelstärke | 60 mg |
| Polyvinylpyrrolidon | 6 mg |
| Natrium-Celluloseglykolat | 4 mg |
| Magnesiumstearat | 2 mg |
| | 300 mg |

Die Bestandteile werden in üblicher Weise zu Tabletten von 300 mg verarbeitet, die jeweils 100 mg erfindungsgemässen Wirkstoff enthalten. Gewünschtenfalls können mit den angegebenen Bestandteilen noch geeignete Mengen bekannter Wirkstoffe vermischt werden.

*Kapseln*

Zusammensetzung:

| | |
|---|---|
| Wirkstoff gemäss der Erfindung | 200 mg |
| Maisstärke | 200 mg |

Die Bestandteile werden entsprechend dem angegebenen Mengenverhältnis gut vermischt und in 400 mg-Portionen in Gelatine-Steckkapseln abgefüllt.

*Beispiel 1*

*1-(6,7-Dimethoxychinazol-4-yl)-semicarbazid*

2,2 g 6,7-Dimethoxy-4-chlor-chinazolin werden in 50 ml absolutem Dimethylformamid gelöst. Dazu gibt man unter Rühren 2,2 g Semicarbazid × HCl und 5 g wasserfreie Pottasche. Man rührt 5 Stunden bei 50° C und saugt den ausgefallenen Niederschlag ab. Er wird mit Wasser verrührt und dann aus Dimethylformamid umkristallisiert. Das Rohprodukt wird in 30%iger Ausbeute erhalten; Fp. 247-249° C.

*Beispiel 2*

*1-n-Butyl-4-(6,7-dimethoxychinazol-4-yl)-semicarbazid-hydrochlorid*

3 g 6,7-Dimethoxy-4-chlor-chinazolin werden zusammen mit 3,5 g 4-n-Butylsemicarbazid in 100 ml n-Amylalkohol 2 Stunden bei 100° C gerührt. Dann wird der ausgefallene Niederschlag abgesaugt, heiss in Alkohol suspendiert und durch Zugabe von 2N Salzsäure auf pH 5 gestellt. Die klare Lösung wird über Kohle filtriert, abgekühlt, abgesaugt und im Umlufttrockenschrank getrocknet. Das in 70%iger Ausbeute erhaltene Hydrochlorid des 4-n-Butyl-4-(6,7-dimethoxychinazol-4-yl)-semicarbazid schmilzt bei 216-217° C.

Entsprechend erhält man

1-n-Butyl-4-(6,7-dimethoxy-2-trifluormethyl-chinazol-4-yl)-semicarbazid-hydrochlorid (Fp. 210-212° C).

1-n-Butyl-4-(6,7-dimethoxy-2-methylchinazol-4-yl)-semicarbazid-hydrochlorid (Fp. 295-297° C).

Analog erhält man ferner die Verbindungen:

R = 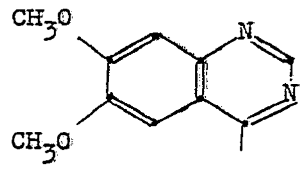

| | Ausbeute (in % d.Th.) | Fp. (°C) |
|---|---|---|
| $R-NH-NH-CO-NH-CH_2-COOCH_3 \times HCl$ | 48 | 214-215 |
| $R-NH-NH-CO-NH-CH_3 \times HCl$ | 89 | 246-247 |
| $R-N(CH_3)-NH-CO-NH-n-C_4H_9 \times HCl$ | 52 | 207-208 |
| $R-N(CH_3)-N(CH_3)-CO-NH-n-C_4H_9 \times HCl$ | 30 | 203-204 |
| $R-NH-NH-CO-NH-C(CH_3)_3 \times HCl$ | 43 | 296-297 |
| $R-NH-NH-CO-NH-C_6H_5 \times HCl$ | 57 | 199 |
| $R-NH-NH-CO-NH-(4-Cl-C_6H_4)$ | 59 | 198-199 |
| $R-NH-NH-CO-NH(3-CH_3-C_6H_4)$ | 55 | 194-195 |
| $R-NH-N(CH_3)-CO-NH-C_4H_9$ | 35 | 210-211 |
| R-NH-NH-CO-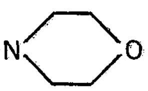 × HCl | 35 | 248-251 |
| $R-NH-NH-CO-N(CH_3)_2 \times HCl$ | 40 | 246-248 |
| R-NH-NH-CO-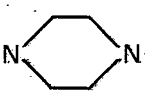-CH₃ × HCl | 36 | 287-288 |
| $R-NH-NH-CO-N(i-C_3H_7)_2$ | 38 | 256-257 |
| $R-NH-NH-CO-NH-(CH_2)_5-CH_3 \times HCl$ | 73 | 213-214 |
| $R-NH-NH-CO-NH-(CH_2)_4-CH_3 \times HCl$ | 49 | 210-211 |
| $R-NH-NH-CO-NH-CH(CH_3)-C_6H_5 \times HCl$ | 53 | 212 |
| $R-NH-NH-CO-NH-CH_2-C_6H_5 \times HCl$ | 79 | 210-211 |
| $R-NH-NH-CO-NH-CH(CH_3)-C_3H_7 \times HCl$ | 56 | 204 |
| $R-NH-NH-CO-NH-CH(C_2H_5)_2 \times HCl$ | 76 | 218-219 |
| $R-NH-NH-CO-NH-CH_2-C(CH_3)_3 \times HCl$ | 84 | 208 |
| $R-NH-NH-CO-NH-CH(CH_3)-CH(CH_3)_2 \times HCl$ | 77 | 197-199 |
| $R-NH-NH-CO-NH-C_2H_5 \times HCl$ | 80 | 226-227 |
| $R-NH-NH-CO-NH-CH(CH_3)_2 \times HCl$ | 81 | 220-221 |
| $R-NH-NH-CO-NH-(CH_2)_7-CH_3 \times HCl$ | 83 | 208-209 |
| R-NH-NH-CO-NH-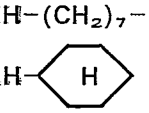 × HCl | 75 | 207-209 |
| R-NH-NH-CO-NH-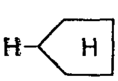 × HCl | 82 | 200-201 |
| $R-NH-NH-CO-NH-n-C_4H_9 \times HCl$ | 69 | 295-297 |

*Beispiel 3*

*1-n-Butyl-4-(6,7-dimethoxychinazolin-4-yl)-semicarbazid-hydrochlorid*

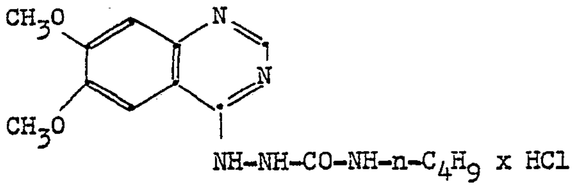

5,12 g 6,7-Dimethoxy-4-hydrazino-chinazolin werden in 200 ml absolutem Acetonitril teilweise gelöst. Dazu gibt man auf einmal 5 ml n-Butylisocyanat. Unter leichtem Temperaturanstieg erhält man kurzzeitig eine Lösung. Man rührt noch 4 Stunden und lässt über Nacht stehen. Der ausgefallene Niederschlag wird abgesaugt, heiss in Ethanol gelöst und mit der berechneten Menge ethanolischer Salzsäure versetzt. Das Hydrochlorid des 1-n-Butyl-4-(6,7-dimethoxy-chinazolin-4-yl)-semicarbazids kristallisiert aus. Nach dem Abkühlen wird abgesaugt und im Umlufttrockenschrank getrocknet. Das in 60%iger Ausbeute erhaltene Endprodukt hat einen Schmelzpunkt von 216-217° C.

*Beispiel 4*

*1-n-Butyl-4-(6,7-dimethoxy-2-methylchinazol-4-yl)-semicarbazid-hydrochlorid*

a) 2,2 g 6,7-Dimethoxy-4-hydrazino-2-methylchinazolin werden in 100 ml absolutem Acetonitril suspendiert. Man gibt auf einmal 2 g Butylisocyanat zu und lässt 3 Stunden bei Raumtemperatur rühren. Die abgesaugte Base wird in Alkohol gelöst und mit der berechneten Menge etherischer Salzsäure versetzt. Das ausgefallene Hydrochlorid (2,3 g, 66% d.Th.) wird abgesaugt und im Trokkenschrank getrocknet (Fp. 295-297° C).

b) Das als Ausgangsmaterial benötigte 6,7-Dimethoxy-4-hydrazino-2-methyl-chinazolin (Fp. 227-229° C) erhält man durch Reaktion von 4-Chlor-6,7-dimethoxy-2-methyl-chinazolin mit überschüssigem Hydrazin in Dimethylformamid.

Nach demselben Vorgehen wird auch das 1-n-Butyl-4-(6,7-dimethoxy-2-trifluormethylchinazol-4-yl)-semicarbazid-hydrochlorid (Fp. 210-212° C) in 73%iger Ausbeute hergestellt.

Entsprechend diesen Beispielen werden synthetisiert:

(R gleich 6,7-Dimethoxychinazolin-4-yl)

| | Ausbeute (in % d.Th.) | Fp. (°C) |
|---|---|---|
| $R-NH-NH-CO-NH-CH_2-COOCH_3 \times HCl$ | 48 | 214-215 |
| $R-NH-NH-CO-NH-CH_3 \times HCl$ | 89 | 246-247 |
| $R-N-NH-CO-NH-C_4H_9 \times HCl$<br>$\quad| $<br>$\quad CH_3$ | 52 | 207-208 |
| $R-N—N-CO-NH-C_4H_9 \times HCl$<br>$\quad| \quad |$<br>$\quad CH_3 \ CH_3$ | 30 | 203-204 |
| $R-NH-NH-CO-NH-C(CH_3)_3 \times HCl$ | 43 | 296-297 |
| $R-NH-NH-CO-NH-C_6H_5 \times HCl$ | 57 | 199 |
| $R-NH-NH-CO-NH-4-Cl-C_6H_4$ | 63 | 198-199 |
| $R-NH-NH-CO-NH-3-CH_3-C_6H_4$ | 70 | 194-195 |
| $R-NH-N-CO-NH-C_4H_9$<br>$\qquad |$<br>$\qquad CH_3$ | 62 | 210-211 |
| $R-NH-NH-CO-NH_2 \times HCl$ | | 247-249 |
| $R-NH-NH-CO-NH-C_2H_5 \times HCl$ | 76 | 226-227 |
| $R-NH-NH-CO-NH-CH(CH_3)_2 \times HCl$ | 87 | 220-221 |
| $R-NH-NH-CO-NH-n-C_5H_{11} \times HCl$ | 50 | 210-211 |
| $R-NH-NH-CO-NH-CH(CH_3)-n-C_3H_7 \times HCl$ | 53 | 204 |
| $R-NH-NH-CO-NH-CH(C_2H_5)_2 \times HCl$ | 84 | 218-219 |
| $R-NH-NH-CO-NH-CH_2-C(CH_3)_3 \times HCl$ | 80 | 208 |

| | Ausbeute (in % d.Th.) | Fp. (°C) |
|---|---|---|
| R–NH–NH–CO–NH–CH($CH_3$)–CH($CH_3$)$_2$ × HCl | 73 | 197-199 |
| R–NH–NH–CO–NH–n–$C_6H_{13}$ × HCl | 70 | 213-214 |
| R–NH–NH–CO–NH–n–$C_8H_{17}$ × HCl | 80 | 208-209 |
| R–NH–NH–CO–NH–$CH_2$–$C_6H_5$ × HCl | 85 | 210-211 |
| R–NH–NH–CO–NH–CH($CH_3$)–$C_6H_5$ × HCl | 59 | 212 |
| R–NH–NH–CO–NH 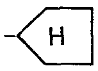 × HCl | 72 | 200-201 |
| R–NH–NH–CO–NH 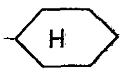 × HCl | 70 | 207-209 |
| R–NH–NH–CO–N($CH_3$)$_2$ × HCl | | 246-248 |
| R–NH–NH–CO–N(i–$C_3H_7$)$_2$ × HCl | | 256-257 |
| R–NH–NH–CO– 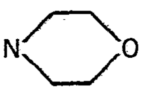 × HCl | | 248-251 |
| R–NH–NH–CO– 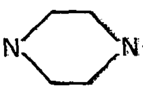 × HCl | | 287-288 |

*Beispiel 5*

*6,7-Dimethoxy-4-(2-morpholinocarbonyl)-hydrazino-chinazolin-hydrochlorid*

2,2 g 6,7-Dimethoxy-4-hydrazino-chinazolin werden in 20 ml Dimethylformamid gelöst. Man fügt 2,76 g wasserfreie Pottasche hinzu und dann unter Rühren tropfenweise 1,7 g Morpholinocarbamidsäurechlorid. Man lässt 2 Stunden bei Raumtemperatur nachreagieren, giesst auf Eiswasser und saugt den ausgefallenen Niederschlag ab. Dieser wird anschliessend in Ethanol gelöst und durch Zugabe von etherischer Salzsäure in das Hydrochlorid überführt. Nach dem Absaugen und Trocknen um Umlufttrockenschrank erhält man das Hydrochlorid des 6,7-Dimethoxy-4-(2-morpholinocarbonyl)-hydrazino-chinazolin in 40%iger Ausbeute; Fp. 248-251° C.

Entsprechend diesem Beispiel werden synthetisiert:

| | Ausbeute (in % d.Th.) | Fp. (°C) |
|---|---|---|
| R–NH–NH–CO– 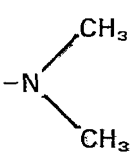 | 30 | 246-248 |
| R–NH–NH–CO– 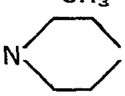 | 35 | 287-288 |
| R–NH–NH–CO–N–(i–$C_3H_7$)$_2$ | 38 | 256-257 |
| R–NH–NH–CO–$NH_2$ × HCl | | 247-249 |
| R–NH–NH–CO–NH–$C_2H_5$ × HCl | 76 | 226-227 |
| R–NH–NH–CO–NH–CH($CH_3$)$_2$ × HCl | 87 | 220-221 |
| R–NH–NH–CO–NH–n–$C_4H_9$ × HCl | | 216-217 |
| R–NH–NH–CO–NH–C($CH_3$)$_3$ × HCl | | 296-297 |
| R–NH–NH–CO–NH–n–$C_5H_{11}$ × HCl | 50 | 210-211 |

| | Ausbeute (in % d.Th.) | Fp. (°C) |
|---|---|---|
| R-NH-NH-CO-NH-$CH(CH_3)$-n-$C_3H_7$ × HCl | 53 | 204 |
| R-NH-NH-CO-NH-$CH(C_2H_5)_2$ × HCl | 84 | 218-219 |
| R-NH-NH-CO-NH-$CH_2$-$C(CH_3)_3$ × HCl | 80 | 208 |
| R-NH-NH-CO-NH-$CH(CH_3)$-$CH(CH_3)_2$ × HCl | 73 | 197-199 |
| R-NH-NH-CO-NH-n-$C_6H_{13}$ × HCl | 70 | 213-214 |
| R-NH-NH-CO-NH-n-$C_8H_{17}$ × HCl | 80 | 208-209 |
| R-NH-NH-CO-NH-$C_6H_5$ × HCl | | 199 |
| R-NH-NH-CO-NH-$CH_2$-$C_6H_5$ × HCl | 85 | 210-211 |
| R-NH-NH-CO-NH-$CH(CH_3)$-$C_6H_5$ × HCl | 59 | 212 |
| R-NH-NH-CO-NH 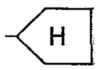 × HCl | 72 | 200-201 |
| R-NH-NH-CO-NH 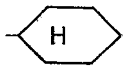 × HCl | 70 | 207-209 |
| R-NH-NH-CO-NH-(3-$CH_3$-$C_6H_4$) × HCl | | 194-195 |
| R-NH-NH-CO-NH-(4-Cl-$C_6H_4$) × HCl | | 198-199 |
| R-N-NH-CO-NH-n-$C_4H_9$ × HCl<br>$CH_3$ | | 207-208 |
| R-NH-N-CO-NH-n-$C_4H_9$ × HCl<br>$CH_3$ | | 210-211 |
| R-N—N-CO-NH-n-$C_4H_9$ × HCl<br>$CH_3$ $CH_3$ | | 203-204 |

*Beispiel 6*

*1-Methyl-4-(6,7-dimethoxychinazolin-4-yl)-semicarbazid-hydrochlorid*

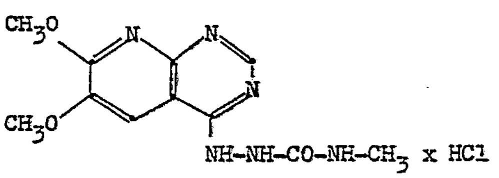

2,2 g 6,7 Dimethoxy-4-hydrazino-chinazolin werden in 100 ml Methanol suspendiert. Nach Zugabe von 1,8 g N-Methylcarbaminsäuremethylester wird 4 Stunden unter Rückfluss gekocht. Die klare Lösung wird abgekühlt, der Niederschlag abgesaugt, nochmals in heissem Methanol gelöst und mit der berechneten Menge etherischer Salzsäure versetzt. Man kühlt und saugt ab und trocknet im Umlufttrockenschrank. Das Hydrochlorid des 1-Methyl-4-(6,7-dimethoxychinazolin-4-yl)-semicarbazids erhält man in 50%iger Ausbeute; Fp. 246-247° C.

Analog erhält man:

R = 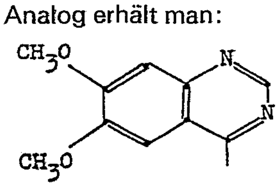

| | Ausbeute (in % d.Th.) | Fp. (°C) |
|---|---|---|
| R-NH-NH-CO-NH-$CH_2$-$COOCH_3$ × HCl | 48 | 214-215 |
| R-N-NH-CO-NH-n-$C_4H_9$ × HCl<br>$CH_3$ | 52 | 207-208 |
| R-N—N-CO-NH-n-$C_4H_9$ × HCl<br>$CH_3$ $CH_3$ | 30 | 203-204 |

| | Ausbeute (in % d.Th.) | Fp. (°C) |
|---|---|---|
| $R-NH-NH-CO-NH-C(CH_3)_3 \times HCl$ | 43 | 296-297 |
| $R-NH-NH-CO-NH-C_6H_5 \times HCl$ | 57 | 199 |
| $R-NH-NH-CO-NH(4-Cl-C_6H_4)$ | 52 | 198-199 |
| $R-NH-NH-CO-NH-(3-CH_3-C_6H_4)$ | 56 | 194-195 |
| $R-NH-N(CH_3)-CO-NH-C_4H_9$ | 35 | 210-211 |
| R-NH-NH-CO-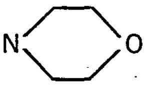 × HCl | 22 | 248-251 |
| $R-NH-NH-CO-N(CH_3)_2 \times HCl$ | 28 | 246-248 |
| R-NH-NH-CO-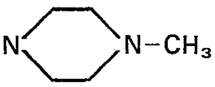 × HCl | 35 | 287-288 |
| $R-NH-NH-CO-N(i-C_3H_7)_2$ | 36 | 256-257 |

| | Fp. (°C) |
|---|---|
| $R-NH-NH-CO-NH_2 \times HCl$ | 247-249 |
| $R-NH-NH-CO-NH-C_2H_5 \times HCl$ | 226-227 |
| $R-NH-NH-CO-NH-CH(CH_3)_2 \times HCl$ | 220-221 |
| $R-NH-NH-CO-NH-n-C_4H_9 \times HCl$ | 216-217 |
| $R-NH-NH-CO-NH-n-C_5H_{11} \times HCl$ | 210-211 |
| $R-NH-NH-CO-NH-CH(CH_3)-C_3H_7 \times HCl$ | 204 |
| $R-NH-NH-CO-NH-CH(C_2H_5)_2 \times HCl$ | 218-219 |
| $R-NH-NH-CO-NH-CH_2-C(CH_3)_3 \times HCl$ | 208 |
| $R-NH-NH-CO-NH-CH(CH_3)-CH(CH_3)_2 \times HCl$ | 197-199 |
| $R-NH-NH-CO-NH-n-C_6H_{13} \times HCl$ | 213-214 |
| $R-NH-NH-CO-NH-n-C_8H_{17} \times HCl$ | 208-209 |
| $R-NH-NH-CO-NH-CH_2-COOCH_3 \times HCl$ | 214-215 |
| $R-NH-NH-CO-NH-CH_2-C_6H_5 \times HCl$ | 210-211 |
| $R-NH-NH-CO-NH-CH(CH_3)-C_6H_5 \times HCl$ | 212 |
| R-NH-NH-CO-NH -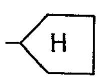 | 200-201 |
| R-NH-NH-CO-NH -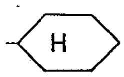 | 207-209 |

Entsprechend erhält man ferner:

1-n-Butyl-4-(6,7-dimethoxy-2-trifluormethyl-chinazol-4-yl)-semicarbazid-hydrochlorid (Fp. 210-212° C).

1-n-Butyl-4-(6,7-dimethoxy-2-methylchinazol-4-yl)-semicarbazid-hydrochlorid (Fp. 295-297° C).

*Beispiel 7*

*1-Methyl-4-(6,7-dimethoxychinazolin-4-yl)-semicarbazid-hydrochlorid*

1,5 g 6,7-Dimethoxy-4-(2-carbethoxy)-hydrazino-chinazolin, durch Umsetzung von 6,7-Dimethoxy-4-hydrazino-chinazolin mit Chlorkohlensäureethylester erhältlich, werden in 30 ml Dioxan gelöst. Nach Zugabe von 2 ml 40%iger Methylamin-Lösung wird 3 Stunden auf 60° C erhitzt. Die ausgefallenen Kristalle werden abgesaugt, heiss in Alkohol suspendiert und mit der berechneten Menge alkoholischer Salzsäure versetzt. Es erfolgt kurzzeitig Lösung. Man kühlt, saugt ab und trocknet. Das Hydrochlorid des 1-Methyl-4-(6,7-dimethoxychinazolin-4-yl)-semicarbazids (Fp. 246-247° C) wird in 20%iger Ausbeute erhalten.

Entsprechend erhält man

1-n-Butyl-4-(6,7-dimethoxy-2-trifluormethyl-chinazol-4-yl)-semicarbazid-hydrochlorid (Fp. 210-212° C).

1-n-Butyl-4-(6,7-dimethoxy-2-methylchinazol-4-yl)-semicarbazid-hydrochlorid (Fp. 295-297° C).

Analog erhält man:

R = 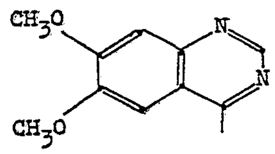

| | Ausbeute (in % d.Th.) | Fp. (°C) |
|---|---|---|
| R-NH-NH-CO-NH-CH$_2$-COOCH$_3$ × HCl | 48 | 214-215 |
| R-N-NH-CO-NH-n-C$_4$H$_9$ × HCl<br>CH$_3$ | 52 | 207-208 |
| R-N—N-CO-NH-n-C$_4$H$_9$ × HCl<br>CH$_3$ CH$_3$ | 30 | 203-204 |
| R-NH-NH-CO-NH-C(CH$_3$)$_3$ × HCl | 43 | 296-297 |
| R-NH-NH-CO-NH-C$_6$H$_5$ × HCl | 57 | 199 |
| R-NH-NH-CO-NH-(4-Cl-C$_6$H$_4$) | 52 | 198-199 |
| R-NH-NH-CO-NH-(3-CH$_3$-C$_6$H$_4$) | 46 | 194-195 |
| R-NH-N-CO-NH-C$_4$H$_9$<br>CH$_3$ | 42 | 210-211 |
| R-NH-NH-CO-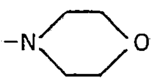 × HCl | 35 | 248-251 |
| R-NH-NH-CO-N(CH$_3$)$_2$ × HCl | 30 | 246-248 |
| R-NH-NH-CO-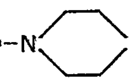-CH$_3$ × HCl | 28 | 287-288 |
| R-NH-NH-CO-N(i-C$_3$H$_7$)$_2$ | 35 | 256-257 |
| R-NH-NH-CO-NH$_2$ × HCl | | 247-249 |
| R-NH-NH-CO-NH-C$_2$H$_5$ × HCl | 76 | 226-227 |
| R-NH-NH-CO-NH-CH(CH$_3$)$_2$ × HCl | 87 | 220-221 |
| R-NH-NH-CO-NH-n-C$_4$H$_9$ × HCl | | 216-217 |
| R-NH-NH-CO-NH-n-C$_5$H$_{11}$ × HCl | 50 | 210-211 |
| R-NH-NH-CO-NH-CH(CH$_3$)-n-C$_3$H$_7$ × HCl | 53 | 204 |
| R-NH-NH-CO-NH-CH(C$_2$H$_5$)$_2$ × HCl | 84 | 218-219 |
| R-NH-NH-CO-NH-CH$_2$-C(CH$_3$)$_3$ × HCl | 80 | 208 |
| R-NH-NH-CO-NH-CH(CH$_3$)-CH(CH$_3$)$_2$ × HCl | 73 | 197-199 |
| R-NH-NH-CO-NH-n-C$_6$H$_{13}$ × HCl | 70 | 213-214 |
| R-NH-NH-CO-NH-n-C$_8$H$_{17}$ × HCl | 80 | 208-209 |
| R-NH-NH-CO-NH-CH$_2$-C$_6$H$_5$ × HCl | 85 | 210-211 |
| R-NH-NH-CO-NH-CH(CH$_3$)-C$_6$H$_5$ × HCl | 59 | 212 |
| R-NH-NH-CO-NH 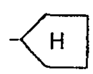 × HCl | 72 | 200-201 |
| R-NH-NH-CO-NH 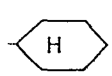 × HCl | 70 | 207-209 |

## Patentansprüche

1. Verbindungen der Formel I

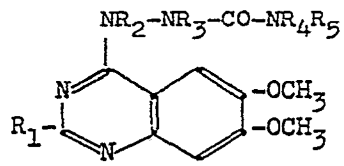

(I)

in der

$R_1$ Wasserstoff, $C_1$-$C_4$-Alkyl oder Trifluormethyl,

$R_2$, $R_3$ und $R_4$ Wasserstoff, Methyl oder Ethyl,

$R_5$ Wasserstoff, gegebenenfalls phenylsubstituiertes $C_1$-$C_8$-Alkyl, $C_3$-$C_7$-Cycloalkyl, $C_1$-$C_4$-Alkoxycarbonylmethyl, Phenyl, Naphthyl, einen durch Methyl, Ethyl, Methoxy, Ethoxy, Trifluormethyl, Fluor, Chlor oder Brom ein- bis dreifach substituierten Phenyl- oder Naphthylrest,

$R_4$ und $R_5$ gemeinsam auch eine ggf. durch O oder $NR_6$ unterbrochene Alkylenkette mit 4 oder 5 Kettengliedern und

$R_6$ ein $C_1$-$C_4$-Alkyl bedeutet, in Form von Säureadditionssalzen und in Form freier Basen.

2. Verbindungen nach Anspruch 1, dadurch gekennzeichnet, dass $R_1$ Wasserstoff oder Methyl, $R_2$ $R_3$ und $R_4$ Wasserstoff, $R_5$ Wasserstoff, $C_3$-$C_5$-Alkyl, $C_5$-$C_6$-Cycloalkyl, Methoxy- oder Ethoxycarbonylmethyl, Phenyl, Chlorphenyl, α- oder β-Naphthyl oder Benzyl, $R_4$ und $R_5$ gemeinsam $-CH_2CH_2-O-CH_2-CH_2-$ oder $-CH_2-CH_2-NCH_3-CH_2-CH_2-$ bedeuten.

3. Arzneimittel, dadurch gekennzeichnet, dass es neben üblichen Hilfs- und/oder Trägerstoffen eine Verbindung nach Anspruch 1 oder 2 enthält.

4. Verwendung von Verbindungen nach Anspruch 1 zur Herstellung von Arzneimitteln für die heilende oder prophylaktische Behandlung von pathologischen Herzzuständen.

5. Verfahren zur Herstellung von Verbindungen der Formel I

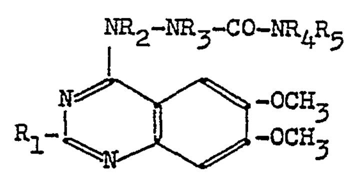

(I)

in der

$R_1$ Wasserstoff, $C_1$-$C_4$-Alkyl oder Trifluormethyl,

$R_2$, $R_3$ und $R_4$ Wasserstoff, Methyl oder Ethyl,

$R_5$ Wasserstoff, gegebenenfalls phenylsubstituiertes $C_1$-$C_8$-Alkyl, $C_3$-$C_7$-Cycloalkyl, $C_1$-$C_4$-Alkoxycarbonylmethyl, Phenyl, einen durch Methyl, Ethyl, Methoxy, Ethoxy, Trifluormethyl, Fluor, Chlor oder Brom ein- bis dreifach substituierten Phenyl- oder Naphthylrest,

$R_4$ und $R_5$ gemeinsam auch eine ggf. durch O oder $NR_6$ unterbrochene Alkylenkette mit 4 oder 5 Kettengliedern und

$R_6$ ein $C_1$-$C_4$-Alkyl bedeutet, in Form von Säureadditionssalzen und in Form freier Basen, dadurch gekennzeichnet, dass man

a) eine Verbindung der Formel II

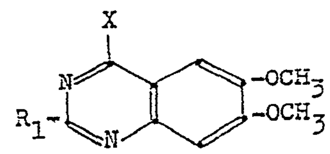

(II)

mit einem Semicarbazid der Formel III

$$HNR_2-NR_3-CO-NR_4R_5 \qquad (III)$$

umsetzt oder dass man

b) eine Verbindung der Formel IV

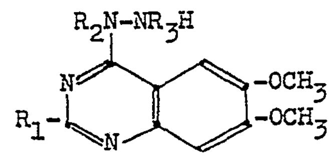

(IV)

mit einem Isocyanat der Formel V

$$OCN-R_5 \qquad (V)$$

umsetzt oder dass man

c) eine Verbindung der Formel IV mit einem Carbamoylchlorid der Formel VI

$$R'_4R'_5-N-COCl \qquad (VI)$$

umsetzt oder dass man

d) eine Verbindung der Formel IV mit einem Carbaminsäureester der Formel VII

$$R_4R_5-N-COOR \qquad (VII)$$

umsetzt oder dass man

e) eine Verbindung der Formel VIII

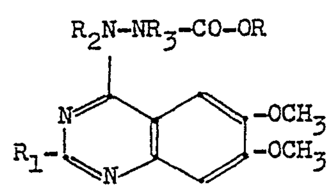

(VIII)

mit einem primären oder sekundären Amin der Formel IX

$$HNR_4R_5 \qquad (IX)$$

umsetzt und dass man gewünschtenfalls nach a) bis e) erhaltene Salze in freie Basen oder Salze anderer Säuren, primär erhaltene freie Basen in Säureadditionssalze überführt.

## Claims

1. Compounds of formula I

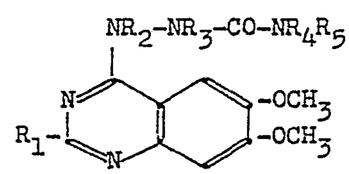

(I)

wherein $R_1$ represents hydrogen, $C_{1-4}$ alkyl or trifluoromethyl, $R_2$, $R_3$ and $R_4$ represent hydrogen,

methyl or ethyl, $R_5$ represents hydrogen, optionally phenyl-substituted $C_{1-8}$ alkyl, $C_{3-7}$ cycloalkyl, $C_{1-4}$ alkoxycarbonyl-methyl, phenyl, naphthyl, a phenyl or naphthyl group mono- to tri-substituted by methyl, ethyl, methoxy, ethoxy, trifluoromethyl, fluorine, chlorine or bromine, $R_4$ and $R_5$ together represent an alkylene chain with 4 or 5 chain members possibly interrupted by O or $NR_6$ and $R_6$ represents a $C_{1-4}$ alkyl, in the form of acid addition salts and in the form of free bases.

2. Compounds as claimed in claim 1, characterized in that $R_1$ represents hydrogen or methyl, $R_2$, $R_3$ and $R_4$ represent hydrogen, $R_5$ represents hydrogen, $C_{3-5}$ alkyl, $C_{5-6}$ cycloalkyl, methoxy- or ethoxycarbonyl-methyl, phenyl, chlorophenyl, α- or β-naphthyl or benzyl, $R_4$ and $R_5$ together represent $-CH_2CH_2-O-CH_2-CH_2-$ or $-CH_2-CH_2-NCH_3-CH_2-CH_2$.

3. Pharmaceutical composition, characterized in that it contains, in addition to conventional excipients and/or carriers, a compound as claimed in claim 1 or 2.

4. Use of compounds as claimed in claim 1 for preparing pharmaceutical compositions for therapeutic or prophylactic treatment of pathological heart conditions.

5. Process for preparing compounds of formula I

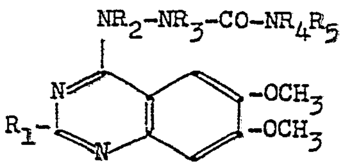
(I)

wherein $R_1$ represents hydrogen, $C_{1-4}$ alkyl or trifluoromethyl, $R_2$, $R_3$ and $R_4$ represent hydrogen, methyl or ethyl, $R_5$ represents hydrogen, optionally phenyl-substituted $C_{1-8}$ alkyl, $C_{3-7}$ cycloalkyl, $C_{1-4}$ alkoxycarbonyl-methyl, phenyl, naphthyl, a phenyl or naphthyl group mono- to tri-substituted by methyl, ethyl, methoxy, ethoxy, trifluoromethyl, fluorine, chlorine or bromine, $R_4$ and $R_5$ together represent an alkylene chain with 4 or 5 chain members possibly interrupted by O or $NR_6$ and $R_6$ represents a $C_{1-4}$ alkyl, in the form of acid addition salts and in the form of free bases, characterized in that

a) a compound of formula II

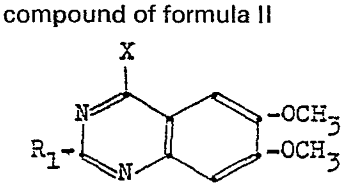
(II)

is reacted with a semicarbazide of formula III

$$HNR_2-NR_3-CONR_4R_5 \quad (III)$$

or

b) a compound of formula IV

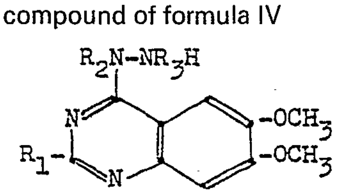
(IV)

is reacted with an isocyanate of formula V

$$OCN-R_5 \quad (V)$$

or

c) a compound of formula IV is reacted with a carbamoyl chloride of formula VI

$$R'_4R'_5-N-COCl \quad (VI)$$

or

d) a compound of formula IV is reacted with a carbamic acid ester of formula VII

$$R_4R_5-N-COOR \quad (VII)$$

or

e) a compound of formula VIII

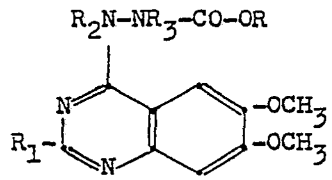
(VIII)

is reacted with a primary or secondary amine of formula IX

$$HNR_4R_5 \quad (IX)$$

and, if desired, salts obtained according to a) to e) are converted into free bases or salts of other acids, whilst free bases obtained in the first place may be converted into acid addition salts.

## Revendications

1. Composés de formule I

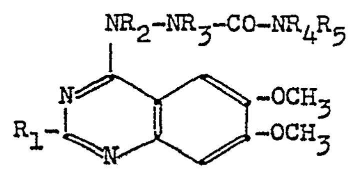
(I)

dans laquelle $R_1$ représente hydrogène, alcoyle en $C_1$-$C_4$ ou trifluorométhyle, $R_2$, $R_3$ et $R_4$ représentent hydrogène, méthyle ou éthyle, $R_5$ représente hydrogène, alcoyle en $C_1$-$C_8$ éventuellement phényl-substitué, cycloalcoyle en $C_3$-$C_7$, alcoxy en $C_1$-$C_4$-carbonylméthyle, phényle, naphtyle, un radical phényle ou naphtyle substitué une à trois fois par méthyle, éthyle, méthoxy, éthoxy, trifluorométhyle, fluor, chlore ou brome, $R_4$ et $R_5$ représentent ensemble également une chaîne alcoylène avec 4 ou 5 membres de chaîne, éventuellement interrompue par O ou $NR_6$, et $R_6$ représente un alcoyle en $C_1$-$C_4$, sous forme de leurs sels d'addition d'acides et sous forme de bases libres.

2. Composés selon la revendication 1, caractérisés en ce que $R_1$ représente hydrogène ou méthyle, $R_2$, $R_3$ et $R_4$ hydrogène, $R_5$ hydrogène, alcoyle en $C_3$-$C_5$, cycloalcoyle en $C_5$-$C_6$, méthoxy-

ou éthoxycarbonylméthyle, phényle, chlorophényle, α- ou β-naphtyle ou benzyle, $R_4$ et $R_5$ ensemble $-CH_2-CH_2-O-CH_2-CH_2-$ ou $-CH_2-CH_2-NCH_3-CH_2-CH_2-$.

3. Médicament, caractérisé en ce qu'il contient, outre des adjuvants et/ou excipients usuels, un composé selon la revendication 1 ou 2.

4. Utilisation de composés selon la revendication 1 pour la préparation de médicaments pour le traitement curatif ou prophylactique d'états cardiaques pathologiques.

5. Procédé pour la préparation de composés de formule I

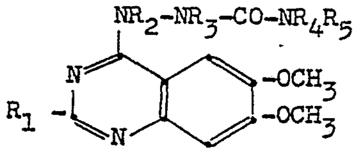

(I)

dans laquelle $R_1$ représente hydrogène, alcoyle en $C_1$-$C_4$ ou trifluorométhyle, $R_2$, $R_3$ et $R_4$ représentent hydrogène, méthyle ou éthyle, $R_5$ représente hydrogène, alcoyle en $C_1$-$C_8$ éventuellement phényl-substitué, cycloalcoyle en $C_3$-$C_7$, alcoxy en $C_1$-$C_4$-carbonylméthyle, phényle, naphtyle, un radical phényle ou naphtyle substitué une à trois fois par méthyle, éthyle, méthoxy, éthoxy, trifluorométhyle, fluor, chlore ou brome, $R_4$ et $R_5$ représentent ensemble également une chaîne alcoylène avec 4 ou 5 membres de chaîne, éventuellement interrompue par O ou $NR_6$, et $R_6$ représente un alcoyle en $C_1$-$C_4$, sous forme de leurs sels d'addition d'acides et sous forme de bases libres, caractérisé en ce que

a) on fait réagir un composé de formule II

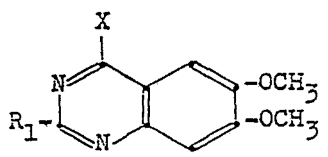

(II)

avec un semi-carbazide de formule III

$$HNR_2-NR_3-CO-NR_4R_5 \qquad (III)$$

ou en ce que

b) on fait réagir un composé de formule IV

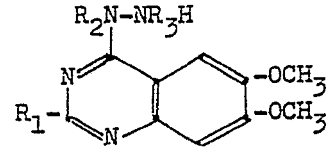

(IV)

avec un isocyanate de formule V

$$OCN-R_5 \qquad (V)$$

ou en ce que

c) on fait réagir un composé de formule IV avec un chlorure de carbamoyle de formule VI

$$R'_4R'_5-N-COCl \qquad (VI)$$

ou en ce que

d) on fait réagir un composé de formule IV avec un ester d'acide carbaminique de formule VII

$$R_4R_5-N-COOR \qquad (VII)$$

ou en ce que

e) on fait réagir un composé de formule VIII

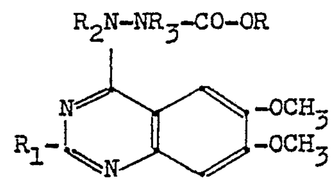

(VIII)

avec une amine primaire ou secondaire de formule IX

$$HNR_4R_5 \qquad (IX)$$

et en ce que, si on le désire, on transforme des sels obtenus selon a) à e) en bases libres ou en sels d'autres acides, des bases libres obtenues en premier en sels d'addition d'acides.